# EUROPEAN PATENT SPECIFICATION

(11) **EP 2 600 938 B1**
(45) Date of publication and mention of the grant of the patent: **19.10.2016**
(21) Application number: 11736116.2
(22) Date of filing: 28.07.2011
(51) Int. Cl.: A61Q 11/00, A61K 8/26, A61K 8/34, A61K 8/46, A61K 8/81, A61K 8/41, A61K 8/86

(54) **ORAL CARE COMPOSITIONS**
MUNDPFLEGEMITTEL
COMPOSITIONS DE PROTECTION ORALE

(30) Priority: 06.08.2010 EP 10172141
(43) Date of publication of application: 12.06.2013
(73) Proprietor: Unilever PLC, London, Greater London EC4Y 0DY (GB); Unilever N.V., 3013 AL Rotterdam (NL)
(72) Inventor: JOINER, Andrew, Wirral Merseyside CH63 3JW (GB); PHILPOTTS, Carole, Jane, Wirral Merseyside CH63 3JW (GB)
(74) Representative: Tansley, Sally Elizabeth
(86) International application number: PCT/EP2011/063028
(87) International publication number: WO 2012/016908

(56) References cited:
- EP-A1- 1 935 395
- WO-A1-01/17494
- WO-A1-98/51270
- GB-A- 2 135 877
- US-A- 4 444 746
- US-A- 6 030 222
- US-A- 6 037 000
- US-A1- 2004 101 492
- US-B1- 6 419 902

## Description

### Field of the Invention

The present invention is concerned with oral care compositions. More particularly, the present invention is concerned with oral care compositions containing lake dyes.

### Background of the Invention

The colour of the teeth is influenced by a combination of their intrinsic colour and the presence of any extrinsic stains that may form on the tooth surface. Extrinsic colour is linked with the adsorption of materials into the acquired pellicle on the surface of enamel, which ultimately cause staining. Factors that influence extrinsic stain formation include poor tooth brushing technique, smoking, dietary intake of coloured foods (e.g. red wine), subject age and the use of certain cationic agents such as chlorhexidine or metal salts like tin and iron.

Consumers have always had a strong desire for white teeth and many individuals are dissatisfied with their current tooth colour. This desire for whiter teeth has given rise to a growing trend in the increased use of tooth whitening products.

Current whitening toothpastes rely on optimised abrasive and chemical components to maximise stain removal and prevention. During brushing, abrasive particles become temporarily trapped between the toothbrush and the stained tooth surface and abrade away the stain. Chemical components may also be used, usually in conjunction with abrasive particles, and include calcium chelators, polymers, surfactants, enzymes, polymers and oxidising agents.

EP 1 935 395 describes a novel optical approach to tooth whitening. On brushing with the toothpaste described in this publication, a blue pigment (in particular blue covarine) is deposited onto the tooth surface, where it is able to change the optical effects of the tooth surface, and enhance the measurement and perception of tooth whiteness. This toothpaste is intended to produce a temporary tooth whitening effect that can be reapplied as frequently as desired, as it contains no harsh chemicals, but is not intended to produce any permanent changes to the colour of the teeth.

The present inventors have now found that lake dyes are particularly effective as temporary tooth whitening agents, and can produce superior temporary tooth whitening effects when used in a context similar to that described in EP 1 935 395.

US 4,444,746 describes the use of lake dyes for imparting stable and effective coloration to visually clear dentifrices. However there is no suggestion that these materials might be effective for delivering a temporary whitening effect to the surface of teeth.

### Summary of the Invention

The present invention provides an oral care composition suitable for delivering a temporary whitening effect to the surface of teeth, the composition comprising:
a continuous phase comprising water or polyhydric alcohol or a mixture thereof;
a tooth surface whitening agent which is dispersed in the continuous phase, and
a deposition aid for the tooth surface whitening agent;
characterised in that the deposition aid is a poly(carboxylic acid) polymer having a molecular weight of at least 50,000 g/mol and an aqueous solubility of at least 10g/L at 25°C and the tooth surface whitening agent is a lake dye formed by fixing a dye onto a particulate inorganic substrate, in which the dye used to form the lake dye is a triarylmethane dye having a blue to green-blue colour with a hue angle in the CIELAB system ranging from 180 to 270 degrees, and in which the amount of lake dye is at least 0.015% by total weight lake dye based on the total weight of the composition.

### Detailed Description of the Invention

### Tooth Surface Whitening Agent

The composition of the invention comprises a tooth surface whitening agent which is dispersed in the continuous phase of the composition.

The tooth surface whitening agent is a lake dye formed by fixing a dye onto a particulate inorganic substrate, in which the dye used to form the lake dye is a triarylmethane dye having a blue to green-blue colour with a hue angle in the CIELAB system ranging from 180 to 270 degrees,

The colour to which the tooth surfaces are whitened depends on the colour of the dye used to form the lake dye. Preferred dyes used to form the lake dye have a blue to green-blue colour with a hue angle in the CIELAB system ranging from 180 to 200 degrees.

Particularly preferred dyes used to form the lake dye are water soluble. The term "water-soluble" in this particular context generally means that the dye has an aqueous solubility of at least 10g/L at 25°C, most preferably at least 100g/L at 25°C (where the solubility is determined in un-buffered distilled water).

Aqueous solutions of particularly preferred dyes used to form the lake dye have a maximum absorbance value in the visible spectrum (Amax) at a wavelength ranging from 550 to 650, more preferably from 600 to 650 nm.

Specific examples of preferred dyes used to form the lake dye include anionic triphenylmethane dyes, and especially diaminotriphenylmethane dyes containing from two to four sulphonate groups, such as those corresponding to general formula (I): 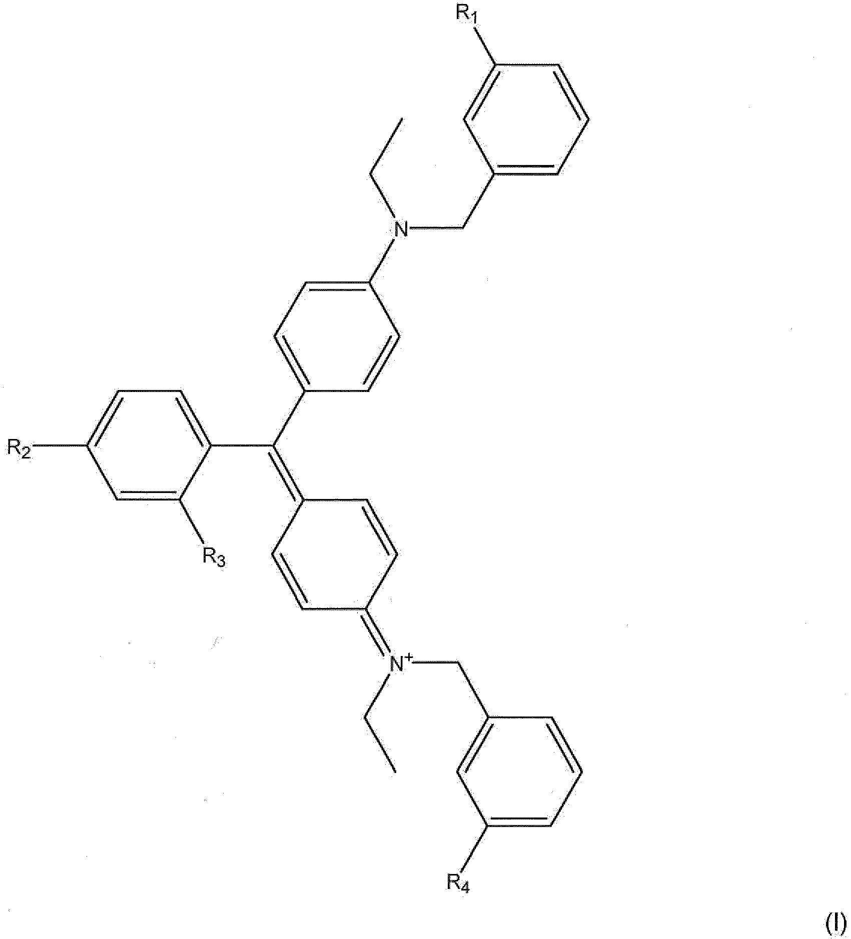 in which R¹ R² ,R³ and R⁴ are monovalent radicals which are each independently selected from hydrogen (-H), hydroxyl
(-OH), halo (e.g. -Cl) and sulphonate (-SO₃⁻) groups, with the proviso that at least two of R¹ to R⁴ are sulphonate groups.

In a preferred example of a dye corresponding to general formula (I), R² is -H and R¹, R³, and R⁴ are sulphonate groups.

The particulate inorganic substrate onto which the dye is fixed is usually a colourless, inert metallic salt of synthetic or mineral origin. Examples of such materials include calcium carbonate, barium sulphate, kaolin (hydrated aluminium silicate), talc (hydrated magnesium silicate) and metal oxides such as titanium dioxide or zinc oxide. For optimum transparency the most preferred substrate is hydrated alumina, also known as alumina trihydrate or aluminium trihydroxide (Al(OH)₃). In a typical dye laking process, an aluminium salt is dissolved in water and an alkali is added to the solution in order to precipitate hydrated alumina. An aqueous solution of the dye is then added to a slurry of the precipitated particles, and a laking reagent (usually aluminium chloride) is added. This causes the dye to precipitate and adsorb onto the hydrated alumina particles, so forming the lake dye.

Typically the dye constitutes 10 to 40% of the lake dye (by total weight dye based on the total weight of the lake dye).

Mixtures of any of the above described materials may also be used.

The most preferred lake dye for use in the invention is formed by fixing a dye of formula (I) in which R² is -H and R¹, R³, and R⁴ are sulphonate groups onto particles of hydrated alumina. This material is commercially available for example as FD&C Blue No.1 Aluminium Lake (C.I. 42090:2). The Colour Index number (C.I.) is taken from the Rowe Colour Index, 3rd edition, Society of Dyers and Colourists, Bradford, England, 1971.

The amount of lake dye (as defined above) in compositions of the invention suitably ranges from 0.015 to 1.0%, preferably from 0.015 to 0.5%, more preferably from 0.02 to 0.1% by total weight lake dye (as defined above) based on the total weight of the composition.

### Deposition Aid

The composition of the invention comprises a deposition aid for the tooth surface whitening agent.

The term "deposition aid" in the context of this invention generally means a material which aids deposition of the tooth whitening agent from the continuous phase of the composition onto the surface of teeth during use of the composition. Use of the composition in the context of this invention typically involves application of the composition to the oral cavity, followed by brushing and/or rinsing).

Suitable deposition aids work by having affinity for both the lake dye (as defined above) and the surface of the teeth.

Preferred deposition aids are able to aid the deposition of the lake dye onto the teeth such that tooth surface whiteness is enhanced by at least 20% and more preferably by at least 100%, in comparison to the value obtained for teeth treated in an equivalent manner with a control formulation using the same amount of lake dye in the absence of the deposition aid.

A convenient measure of enhanced tooth surface whiteness is delta b* measured using a chromameter. A negative value of delta b* indicates a yellow to blue colour shift which has been shown to be one of the primary drivers of tooth surface whiteness as perceived by the consumer.

Accordingly, preferred deposition aids are able to aid the deposition of the lake dye onto the teeth such that the negative value of delta b* is augmented by at least 20% and more preferably by at least 100%, in comparison to the value obtained for teeth treated in an equivalent manner with a control formulation using the same amount of lake dye in the absence of the deposition aid.

A simple empirical model for establishing the effect of the deposition aid is as follows:
Polished hydroxyapatite discs are first placed in sterile human saliva for 2 hours to allow a pellicle to form. The discs are then rinsed in water and baseline colour measurements made (using, for example, a Minolta chromameter CR300). The discs are then brushed with either (a) a suspension of the lake dye in water; or (b) a suspension of the lake dye in water at the same concentration as in (a), together with the deposition aid. The brushing is best performed using a brushing machine. Following rinsing, the colour of the discs is then re-measured and the change in delta b* is recorded for both treatment (a) and treatment (b). From a comparison of these data, the effect of the deposition aid is readily seen.

Suitable deposition aids for use in the invention will generally dissolve or disperse in water at a temperature of 25°C.

Deposition aids for use in the invention are water soluble. The term "watersoluble" in this particular context generally means that the deposition aid has an aqueous solubility of at least 10g/L at 25°C, and more preferably at least 30g/L at 25°C (where the solubility is determined in un-buffered distilled water). It is particularly preferable that the deposition aid remains water soluble after drying, so that it can be re-dissolved. This prevents undesirable build up of the deposition aid on the teeth after repeated usage of the composition.

Suitable deposition aids for use in the invention include polymeric materials, preferably polymeric materials which are water soluble as defined above. Polymeric materials for use as deposition aids in the invention may be naturally or synthetically-derived, and may be ionic or nonionic in nature.

Polymeric materials for use in the invention are high molecular weight. The term "high molecular weight" in this particular context means that the polymeric material has a molecular weight of at least 50,000, more preferably at least 500,000 g/mol. A suitable method to determine the molecular weight of such polymeric materials is gel permeation chromatography against a polyethylene glycol standard.

Polymeric material for use as deposition aids in the invention includes watersoluble, high molecular weight polymers having anionic side groups along the polymer main chain and
include poly(carboxylic acid) polymers. Poly (carboxylic acid) polymers are typically polymers which include -COOH groups in
their structure, or groups which are derived from -COOH groups such as salt, ester or anhydride groups.

For example, the poly(carboxylic acid) polymers may include:

-[C(R¹)(COOH)-]-

units in their structure, in which R¹ is selected from hydrogen, C₁₋₃ alkyl, C₁₋₃ alkoxy or C₁₋₃ hydroxyalkyl. Preferably R¹ is hydrogen.

A preferred type of poly (carboxylic acid) polymer includes adjacent:

-[C(R¹)(COOH)-]-

units in its structure (where R¹ is as defined above), for example polymers based on maleic acid, which typically include:

-{-CH(COOH)-CH(COOH)-]-

units, and/or salts or esters of such units, or such units in anhydride form in which -COOH groups on adjacent carbon atoms are cyclised to form a ring system.

For example, the poly(carboxylic acid) polymers may comprise units with pairs of carboxylic acid groups on adjacent polymer chain carbon atoms, for example polymers comprising:

-[-C(R¹)(R²)-C(R³)(R⁴)-C(R⁵)(COOH)- C(R⁶)(COOH)-]-

units in its structure (and/or salts or esters of such units, or such units in anhydride form in which -COOH groups on adjacent carbon atoms are cyclised to form a ring system); in which R¹,R²,R³,R⁴,R⁵ and R⁶ are each independently selected from hydrogen, C₁₋₃ alkyl or C₁₋₃ alkoxy. Preferably R¹ and R² are hydrogen, R³ is hydrogen and R⁴ is methoxy and R⁶ and R⁶ are hydrogen.

Such a poly(carboxylic acid) polymer may be described as the polymer based on a copolymer of methyl vinyl ether and maleic anhydride, and is commercially available for example under the trade name Gantrez®.

A particularly preferred example of such a polymer comprises:

-[-CH₂-CH(OCH₃)-CH(COOH)-CH(COOH)-]-

units in its structure, in which the -COOH groups are in free acid form. Such polymers may be linear or cross-linked. More preferably the polymer is linear. Polymers of this type are commercially available for example under the trade name Gantrez® S. Most preferably such a polymer has a molecular weight of at least 500,000 g/mol (e.g. Gantrez® S-96), ideally at least 1,000,000 g/mol (e.g. Gantrez® S-97).

Alternative polymers which may be used comprise the units as described above in anhydride form, i.e. in which the two adjacent -COOH groups are cyclised to form a ring system. Such polymers are commercially available under the tradename Gantrez® AN, e.g. Gantrez® AN-119, Gantrez® AN-803, Gantrez® AN-139 and Gantrez® AN-169.

Other alternative polymers which may be used comprise the units as described above in partial salt form, for example in which some of the free -COOH groups are converted into a metal salt of a Group I or Group II metal such as either sodium or calcium, or a mixed sodium-calcium salt. Such polymers are commercially available under the tradename Gantrez® MS, e.g. Gantrez® MS-955.

Other alternative polymers which may be used comprise the units as described above in partial ester form, for example in which some of the free -COOH groups are esterified with C₁₋₆ alkyl, e.g. ethyl or n-butyl. Such polymers are commercially available under the tradename Gantrez® ES, e.g. Gantrez® ES-225 or Gantrez® ES-425.

Mixtures of any of the above described materials may also be used.

The amount of deposition aid (as defined above) in compositions of the invention suitably ranges from 0.001 to 5.0%, preferably from 0.005 to 4.0%, more preferably from 0.01 to 2.0% by total weight deposition aid (as defined above) based on the total weight of the composition.

### Product Form

The composition of the invention comprises a continuous phase comprising water or polyhydric alcohol or a mixture thereof.

Examples of suitable product forms for compositions of the invention include dentifrices, mouthwashes, chewing gums and lozenges.

A preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice composition according to the invention will generally contain further ingredients to enhance performance and/or consumer acceptability such as abrasive cleaning agent, binder or thickening agent, and surfactant.

For example, a dentifrice will usually comprise an abrasive cleaning agent in an amount of from 3 to 75% by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice will usually contain a binder or thickening agent in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of C₈ to C₁₈ alkyl sulphates (for example sodium lauryl sulphate), C₈ to C₁₈ alkyl sulphosuccinates (for example dioctyl sodium sulphosuccinate), C₈ to C₁₈ alkyl sulphoacetates (such as sodium lauryl sulphoacetate), C₈ to C₁₈ alkyl sarcosinates (such as sodium lauryl sarcosinate), C₈ to C₁₈ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Another preferred type of product form in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition according to the invention will usually contain an aqueous continuous phase. The amount of water generally ranges from 70 to 99% by weight based on the total weight of the mouthwash.

A mouthwash composition according to the invention will generally contain further ingredients to enhance performance and/or consumer acceptability, such as the humectants and surfactants mentioned above for dentifrices. The amount of humectant generally ranges from 5 to 20% by weight based on the total weight of the mouthwash and the amount of surfactant generally ranges from 0.1 to 5% by weight based on the total weight of the mouthwash.

Compositions of the present invention (such as in particular dentifrices or mouthwashes) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

The invention is further illustrated with reference to the following, non-limiting Examples.

### EXAMPLE

### Formulations

Paste compositions having the ingredients shown in Table 1 below were used to prepare test pastes.

All ingredients are expressed by weight percent of the total formulation, and as level of active ingredient.

**Table 1**

| Ingredient | Blue Formulation A | Blue Formulation 1 | Sheath Formulation |
|---|---|---|---|
| Water | 7.955 | 6.497 | 9.33 |
| Sorbitol | 66 | 66 | 66 |
| PEG 32 | 2 | 2 | 2 |
| Sodium fluoride | 0.32 | 0.32 | 0.32 |
| Sodium saccharin | 0.20 | 0.20 | 0.2 |
| Trisodium phosphate | 1.1 | 1.1 | 0 |
| Gantrez® S-97 | 1.0 | 1.0 | 0 |
| Thickening silica | 6 | 6 | 9 |
| Abrasive silica | 11 | 11 | 9.5 |
| Cellulose gum | 0.7 | 0.7 | 0,5 |
| Sodium lauryl sulphate | 1.8 | 1.8 | 1.8 |
| Flavour | 1.3 | 1.3 | 1.3 |
| Mica | 0 | 0 | 0.05 |
| Blue Covarine (C_ 1.741 CO); 40% a.i. | 0.625 | 0 | 0 |
| FD&C Blue No.1 Aluminium Lake (C.I. 42090:2); 12% a.i. | 0 | 2.083 | 0 |
| Total | 100 | 100 | 100 |

Test pastes were prepared, as follows:
Comparative Example A (not according to the invention): 10% w/w of Blue Formulation A and 90% w/w of Sheath Formulation.
Example 1 (according to the invention): 10% w/w of Blue Formulation 1 and 90% w/w of Sheath Formulation.

Each paste was made into an aqueous slurry (paste:water, 1:2), to give a final concentration of blue colorant in each case of 0.025% w/w, and evaluated for whitening performance as follows:

### Method

Hydroxyapatite (HA) discs were polished using P1200 grit silicon carbide paper and thoroughly rinsed with water. The discs were immersed in sterilised whole human saliva for a minimum of two hours to allow a pellicle to form. The HA discs were removed from the saliva, rinsed with water and the baseline colour of the discs were measured with a colorimeter in the CIELAB mode. The HA discs were then placed in a brushing machine and 10ml of the test slurry added per disc. The discs were brushed for 1 minute under a brushing load of 375 g. After brushing the HA discs were rinsed in water and the colour of the discs re-measured. Changes in the colour co-ordinates L*, a* and b* and whiteness index (WIO) were calculated. The WIO index is an index which has been optimised specifically for the evaluation of whiteness in teeth (as described in Journal of Dentistry, Volume 36, Supplement 1, 2008, pages 2 to 7).

### Results

The changes in L*, a*, b* and whiteness (WIO) are shown in Table 2 below. For changes in WIO, the product differences were of statistical significance (p<0.05, Tukey-Kramer).

**Table 2**

| Mean changes in colour parameters (s.d.) | | | | |
|---|---|---|---|---|
| Formulation | Delta L* | Delta a* | Delta b* | Delta WIO |
| Comparative Example A | -2.40 (0.60) | -1.37 (0.31) | -6.74 (0.84) | 11.09 (1.72) |
| Example 1 | -2.75 (0.62) | -6.17 (0.91) | -7.00 (0.53) | 17.32 (1.21) |

The results demonstrate the superior temporary tooth whitening effect obtained with the formulation of Example 1, compared with Comparative Example A which uses the preferred blue pigment of EP 1 935 395.

## Claims

1. An oral care composition suitable for delivering a temporary whitening effect to the surface of teeth, the composition comprising:
a continuous phase comprising water or polyhydric alcohol or a mixture thereof; a tooth surface whitening agent which is dispersed in the continuous phase, and a deposition aid for the tooth surface whitening agent;
**characterised in that** the deposition aid **is a poly(carboxylic acid) polymer having a molecular weight of at least 50,000 g/mol and an aqueous solubility of at least 10g/L at 25°C** and the tooth surface whitening agent is a lake dye formed by fixing a dye onto a particulate inorganic substrate, in which the dye used to form the lake dye is a triarylmethane dye having a blue to green-blue colour with a hue angle in the CIELAB system ranging from 180 to 270 degrees, and in which the amount of lake dye is at least 0.015% by total weight lake dye based on the total weight of the composition.

2. An oral care composition according to claim 1, in which the dye used to form the lake dye has a hue angle in the CIELAB system ranging from 180 to 200 degrees.

3. An oral care composition according to claim 1 or claim 2, in which the particulate inorganic substrate onto which the dye is fixed is hydrated alumina.

4. An oral care composition according to any preceding claim, which is in the form of a dentifrice or a mouthwash.

5. The use of a lake dye formed by fixing a dye onto a particulate inorganic substrate, in which the dye used to form the lake dye is a triarylmethane dye having a blue to green-blue colour with a hue angle in the CIELAB system ranging from 180 to 270 degrees, for delivering a temporary whitening effect to the surface of teeth.

## Patentansprüche

1. Mundpflegezusammensetzung, die geeignet ist, einen temporären Aufhelleffekt auf der Oberfläche von Zähnen zu liefern, wobei die Zusammensetzung umfasst:
eine kontinuierliche Phase, die Wasser oder mehrwertigen Alkohol oder eine Mischung davon umfasst, ein Aufhellungsmittel für die Zahnoberfläche, das in der kontinuierlichen Phase dispergiert ist, und ein Abscheidungshilfsmittel für das Aufhellmittel für die Zahnoberfläche,
**dadurch gekennzeichnet, dass** das Abscheidungshilfsmittel ein Poly(carbonsäure)polymer mit einem Molekulargewicht von mindestens 50.000 g/Mol und einer Wasserlöslichkeit von mindestens 10 g/l bei 25°C aufweist und das Aufhellmittel für die Zahnoberfläche ein Lackfarbstoff ist, der durch Fixieren eines Farbstoffs auf einem teilchenförmigen anorganischen Substrat gebildet wurde, wobei der zur Bildung des Lackfarbstoffs verwendete Farbstoff ein Triarylmethanfarbstoff mit einer blauen bis grün-blauen Farbe mit einem Farbtonwinkel im CIELAB-System in dem Bereich von 180 bis 270 Grad Celsius ist und wobei die Menge des Lackfarbstoffs mindestens 0,015 Gew.-% an gesamtem Lackfarbstoff, bezogen auf das Gesamtgewicht der Zusammensetzung, beträgt.

2. Mundpflegezusammensetzung nach Anspruch 1, in welcher der zur Bildung des Lackfarbstoffs verwendete Farbstoff einen Farbtonwinkel im CIELAB-System in dem Bereich von 180 bis 200 Grad aufweist.

3. Mundpflegezusammensetzung nach Anspruch 1 oder Anspruch 2, in welcher das teilchenförmige anorganische Substrat, auf dem der Farbstoff fixiert ist, hydriertes Aluminiumoxid ist.

4. Mundpflegezusammensetzung nach irgendeinem vorhergehenden Anspruch, das in Form eines Zahnputzmittels oder eines Mundwassers vorliegt.

5. Verwendung eines Lackfarbstoffs, der durch Fixieren eines Farbstoffs auf einem teilchenförmigen anorganischen Substrat gebildet ist, in welcher der zur Bildung des Lackfarbstoffs verwendete Farbstoff ein Triarylmethanfarbstoff mit einer blauen bis grün-blauen Farbe mit einem Farbtonwinkel im CIELAB-System in dem Bereich von 180 bis 270 Grad Celsius ist, zum Liefern eines temporären Aufhelleffekts auf der Oberfläche von Zähnen.

## Revendications

1. Composition pour soins bucco-dentaires capable d'exercer un effet blanchissant temporaire sur la surface des dents, la composition comprenant :
une phase continue comprenant de l'eau ou un alcool polyhydrique ou un mélange des deux ; un agent de blanchiment de la surface des dents qui est dispersé dans la phase continue, et un auxiliaire de dépôt pour l'agent de blanchiment de la surface des dents ;
**caractérisée en ce que** l'auxiliaire de dépôt est un polymère d'acide polycarboxylique ayant un poids moléculaire d'au moins 50 000 g/mol et une solubilité aqueuse d'au moins 10 g/L à 25 °C et **en ce que** l'agent de blanchiment de la surface des dents est un colorant-laque formé par fixation d'un colorant sur un substrat inorganique particulaire, dans laquelle le colorant utilisé pour former le colorant-laque est un colorant au triarylméthane ayant une couleur bleue à vert-bleu et un angle de teinte dans le système CIELAB allant de 180 à 270 degrés, et dans laquelle la quantité de colorant-laque est d'au moins 0,015 % en poids total de colorant-laque sur la base du poids total de la composition.

2. Composition pour soins bucco-dentaires selon la revendication 1, dans laquelle le colorant utilisé pour former le colorant-laque a un angle de teinte dans le système CIELAB allant de 180 à 200 degrés.

3. Composition pour soins bucco-dentaires selon la revendication 1 ou la revendication 2, dans laquelle le substrat inorganique particulaire sur lequel le colorant est fixé est une alumine hydratée.

4. Composition pour soins bucco-dentaires selon l'une quelconque des revendications précédentes, qui est sous la forme d'un dentifrice ou d'un bain de bouche.

5. Utilisation d'un colorant-laque formé par fixation d'un colorant sur un substrat inorganique particulaire, dans laquelle le colorant utilisé pour former le colorant-laque est un colorant au triarylméthane ayant une couleur bleue à vert-bleu et un angle de teinte dans le système CIELAB allant de 180 à 270 degrés, pour exercer un effet blanchissant temporaire sur la surface des dents.
